# EUROPEAN PATENT SPECIFICATION

(11) **EP 2 405 740 B1**
(45) Date of publication and mention of the grant of the patent: **23.12.2015**
(21) Application number: 10709826.1
(22) Date of filing: 12.03.2010
(51) Int. Cl.: G01N 33/68, A01K 45/00, A61K 49/00

(54) **METHOD FOR AVIAN SEX DETERMINATION**
VERFAHREN ZUR GESCHLECHTSBESTIMMUNG VON VÖGELN
Procédé de détermination du sexe aviaire

(30) Priority: 13.03.2009 EP 09155184
(43) Date of publication of application: 18.01.2012
(73) Proprietor: Van de Ven Beheer B.V., 5521 DZ Eersel (NL)
(72) Inventor: DECUYPERE, Eddy, B-3001 Heverle (BE); FEY, Frank, NL-5670 AA Nuenen (NL)
(74) Representative: Derks, Wilbert
(86) International application number: PCT/EP2010/053210
(87) International publication number: WO 2010/103111

(56) References cited:
- WO-A1-2004/016812
- WO-A2-2006/078499
- RAAB U ET AL: "Endoglin is expressed in the chicken vasculature and is involved in angiogenesis" FEBS LETTERS, ELSEVIER, AMSTERDAM, NL, vol. 459, no. 2, 8 October 1999 (1999-10-08), pages 249-254, XP004260356 ISSN: 0014-5793
- MORRISH BRONWYN C ET AL: "Vertebrate sex determination: Many means to an end" REPRODUCTION (CAMBRIDGE), vol. 124, no. 4, October 2002 (2002-10), pages 447-457, XP002569740 ISSN: 1470-1626
- KOYAMA YOSHIYUKI ET AL: "Sequence analysis of full-length cDNA of sex chromosome-linked novel gene 2d-2F9 in Gallus gallus" BIOSCIENCE BIOTECHNOLOGY AND BIOCHEMISTRY, vol. 71, no. 2, February 2007 (2007-02), pages 561-570, XP002569741 ISSN: 0916-8451

## Description

### Field of the invention

The invention relates to a method and apparatus for pre-hatch avian embryo sex determination. In particular, the invention relates to a non-invasive method and apparatus for determining the sex of avian species while in the egg and allowing to sort the eggs into groups consisting primarily of either male or female embryos. The invention uses a non-invasive in-ovo measurement method to make the sex determination. In-ovo means measurement in the egg without having to take a sample from the egg.

### Description of the Related Art

Sex separation is an important aspect for all avian species production, but in particular for the broiler and essentially all egg layer and turkey production. For broilers and turkeys, sex separation allows a better suited management and feeding according to the needs of both sexes, which are somewhat different than the unisex rearing as done now in most cases. Essentially all commercial hatcheries of pullets that will become table egg laying hens use sex separation of flocks. Male chickens are culled at the hatchery, whereas female chickens are evidently destined for egg production.

Currently, there are three methods available for sexing poultry. Day-old chicks can be sexed either by vent/cloaca sexing, or feather sexing methods. Alternatively, male and female chicks can be reared together until secondary sex characteristics become apparent, then the chicks can be separated based on sex. Vent/cloaca sexing relies on the visual identification of sex, based on the appearance of sex related anatomical structures. Feather sexing is based on feather characteristics that differ between male and female chicks, for example down colour pattern, and rapid/slow rate of growth of the wing feathers. The third method relies on the appearance of natural secondary sex characteristics, for example in males the combs and wattles will become larger than those on females.

The vent/cloaca sex determination of day-old chicks is difficult and expensive. Identifying the sex of a bird requires highly skilled personnel. While easier to perform, feather sexing has the disadvantage of being limited to specific genetic crosses of birds. Sexing by secondary sex characteristics is the easiest method to perform but has the disadvantage of requiring birds of both sexes to be reared together for the first weeks after hatch which because of feed costs and feed conversion considerations can be more expensive to the hatchery than the expense of vent/cloaca sexing.

Most importantly, because of the increasing optimization of meat production on one hand and egg production on the other hand, the male pullets of chicken breeds that are optimized for egg productions are no longer suitable or economically attractive for meat production. Therefore, over 500 million male chicks are destroyed every year in the US and Europe only by gassing, electrocution or shredding. This is not only an economic problem, but it has also increasingly become an ethical problem.

Clearly, the commercial hatchery industry has a need for a method that would allow birds to be sorted by sex at very early stage that does not rely on highly skilled individuals or on using specific genetic crosses of birds. Several methods have recently been developed.

In US6512839 it is described that the sex of a hatchling is determined by ultrasound vent or cloaca scanning techniques. Although this may automate and reduce costs of the sex determination process, it still has the disadvantage that it is a post-hatching technique and therefore that the eggs must be hatched and that male chicks need to be destroyed.

In US6029080 a method and apparatus is described for in-ovo determination of the sex of avian species comprising NMR imaging on the intact egg (non-invasive) to determine the presence of male or female sex organs in the embryo of the hatching eggs. The NMR signals are detected and converted to an image of the sex organs, which is interpreted by an image processing computer to identify at the sex of the embryo in the egg. Although it is a non-invasive technique, it has certain disadvantages. A particular disadvantage of this method is that it can only be applied in a late stage of development of embryo were the sex organs have been developed to the extent that they can be recognized. Apart from the fact that this is a waste of hatching capacity, the disadvantage is that the male eggs cannot be used for other purposes and that at this stage of development of the embryo the destruction may still raise ethical problems. Furthermore, the method is economically less attractive, first of all because NMR detection apparatus, certainly in combination with the imaging equipment, is very expensive and the technique is very complicated and because the eggs are preferably cooled to prevent movement of the embryo.

In US7167579 it is described to determine the sex of an egg by taking an image of the egg, generating 2-dimensional contour image data from the image, extracting parameters from the contour image data and determining the sex of the chicken egg using said extracted parameters. It is considered that this method is not sufficiently reliable.

In WO2004/01681 the sex of an egg of an avian subject is determined by taking a sample of an egg, preferably from the allantoic fluid or amniotic fluid, and determining the presence of certain DNA sequences by hybridizing with a specified nucleic acid probe. The analysis of the hybridised sample includes a PCR amplification procedure.

Although this method is accurate and sufficiently reliable, it is highly elaborate, slow and expensive and therefore economically unattractive, but most important it is applied in a rather late stage of development where the amnion is already developed. The method is invasive, meaning that a sample must be taken from the egg, which significantly increases the cost and increases the risk of creating infections and aberrations. Further, the method relies on very expensive and time-consuming nucleic acid amplification techniques like PCR. This means that this sex determination method can never have a high production capacity and is economically unattractive for commercial hatcheries.

The document WO2004/01681 also mentions the use of (monoclonal) antibodies to a peptide (and derivatives, fragments and synthetic constructs thereof capable of binding to the polypeptide) that is coded for by a sex specific DNA sequence. The method comprises contacting a sample extracted from said avian subject with the (preferably detectably labelled) antibody, and is ex-ovo. The document also mentions synthetic antibody constructs comprising a covalently linked moiety which provides the molecule with some desirable property in addition to antigen binding, for example a label (a detectable label, such as fluorescent or radioactive label) or a phannaceutically active agent. Example 9 describes a method of producing monoclonal antibodies for identifying FAF peptides.

### Brief description of the invention:

The problem underlying the invention is to provide a method and apparatus for pre-hatch avian embryo sex determination that can be applied in an early stage of development of the embryo, that is not invasive, meaning that no sample needs to be taken from the egg, and that is relatively easy, inexpensive and fast such that it is economically attractive for commercial hatcheries.

This problem is solved according to the invention by a method for pre-hatch determination of the sex of an embryo in an avian egg, comprising the steps of
a. introducing into the egg an antibody designed to match with a sex specific antigen on the embryo, which antibody is labelled,
b. allowing the labelled antibody to migrate to and bind with the sex specific antigen on the embryo,
c. detecting binding of the labelled antibodies on the embryo using detection means positioned outside of the egg.

When incubating a fertilised egg, sex specific antigens on the embryo are formed at a very early stage of the development of the embryo in the blastodisc, which is located at one side of the egg yolk. The labelled antibodies will bind and a higher concentration of the labelled antibodies will be present, in or near the embryo. Labelled herein means having a functional group that acts as a label to allow detection of the antibody and the antibody-antigen combination. Male eggs can be identified by detecting the local concentration of labelled antibodies bound on the embryo or, in case labelled antibodies are used specific for female antigens, by the absence of such binding. The inventors have found a method for detecting the presence of such sex specific antigens using selected antibodies and detecting binding by detection means positioned outside of the egg that is a non-invasive, i.e. can be done without taking a sample from the egg.

### Brief description of the drawings:

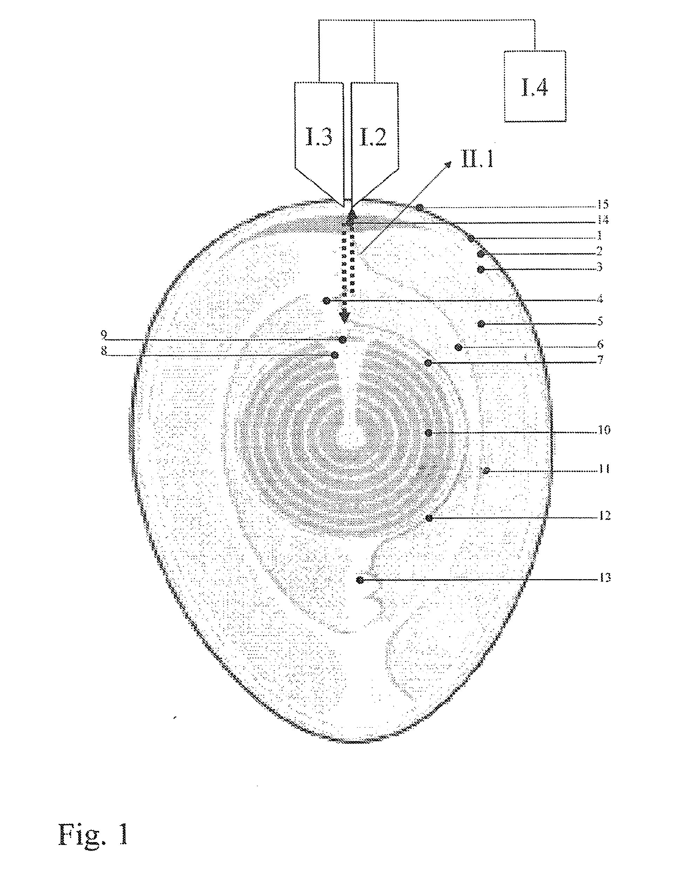

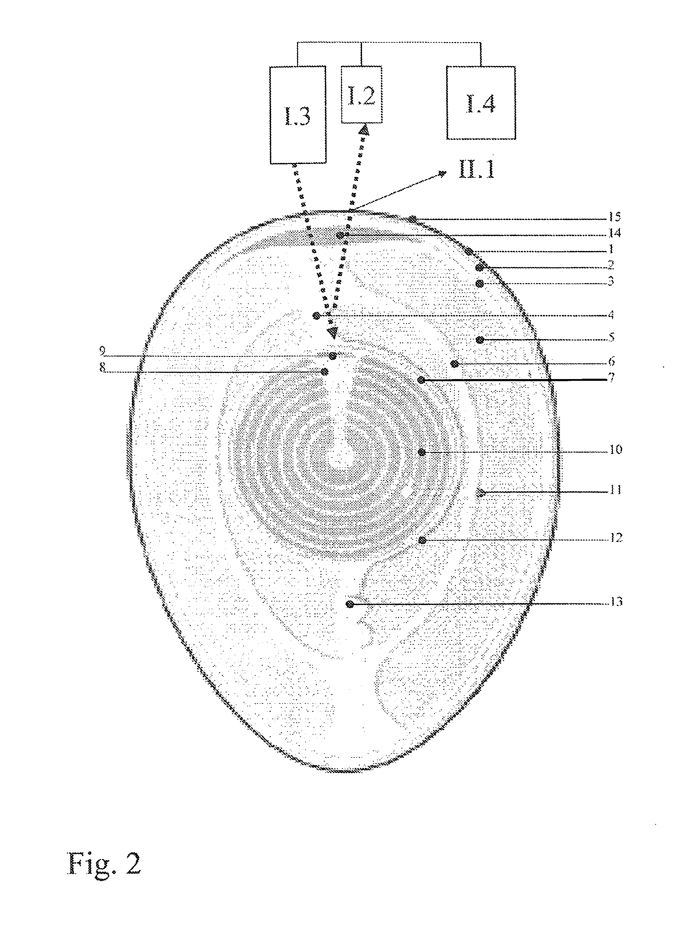

The features and advantages of the invention will be appreciated upon reference to the Figures 1 and 2.
Figure 1 illustrates an egg positioned with the broad end upwards indicating: 1. Eggshell, 2. Outer membrane, 3. Inner membrane, 4. Chalaza, 5. Exterior albumen (outer thin albumen), 6. Middle albumen (inner thick albumen), 7. Vitelline membrane, 8. Nucleus of Pander, 9. Germinal disk (also referred to as blastoderm or blastodisc), 10. Yellow yolk, 11. White yolk, 12. Internal albumen, 13. Chalaza, 14. Air cell and 15. Cuticula and further illustrating detection means I comprising light source I.3 and detector 1.2 penetrating the cuticula (15), eggshell (1) and outer membrane (2), but not inner membrane (3) ending in the air cell (14), detector 1.2 measuring the emitted light (II.1) from labelled antibodies attached to the embryo, 1.4 processes the data and determines if a local high concentration of labelled antibodies is present indicating the female embryo.
Figure 2 illustrates an egg with detection means I comprising light source I.3 positioned outside the egg and a detector 1.2 also positioned outside the egg for measuring emitted light (II.1) from labelled antibodies near the embryo or blastodisc and evaluation means 1.4 to determine and evaluate from the measured value binding of sex specific labelled antibodies indicative of the presence of an embryo.

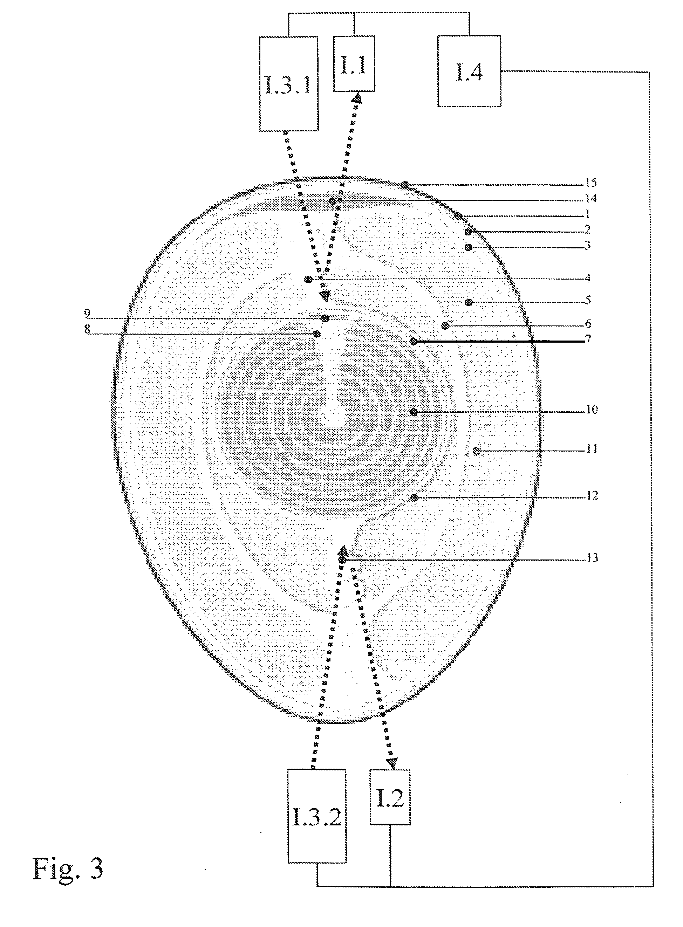

Figure 3 illustrates an egg with detection means I comprising two light sources I.3.1 and 1.3.2 positioned outside the egg at the top and bottom of the egg (along the central long axis) and a detectors 1.1 and 1.2 also positioned outside the egg for measuring emitted light from labelled antibodies. 1.4 is used to determine and evaluate the ratio or difference of the measured values of the first and second detector to establish binding of sex specific labelled antibodies near the embryo indicative of the presence of an embryo.

### Detailed description of the invention

The method comprises the step of introducing into the egg an antibody designed to match with a sex specific antigen on the embryo, which antibody is labelled. The antibody can be introduced into the egg in a variety of methods known in the art, preferably by injection into the egg, preferably by micro-injection using a needle or by micro-fluid injection using a high-pressure fluid jet or by osmosis.

A suitable injection method is described in WO2006/078499. This document describes a method to detect whether a pocket in a flat contains an egg or not or to detect injection tool position relative to the egg using a sensor (optical camera). This is part of an apparatus for injecting a substance in an egg or extracting substance from an egg, which is useful in the method according to the invention. Examples of substances injected include vaccines, antibiotics and vitamins.

The term labelled antibody is defined as an antibody having a functional group capable of detection. Preferably the labelled antibody can be detected by absorption or emission of light. Preferably the functional group is a luminescent group, more preferably it is a photo-luminescent group that can be excited by light to emit light at a characteristic wavelength, most preferably a fluorescent or phosphorescent group. Alternatively, the functional group can be a magnetic nanoparticle, capable of being detected by magnetic or electric detection means.

The labelled antibody can be injected directly into the albumen or can be injected into the air cell and allowed to diffuse through the inner shell membrane into the albumen. The advantage of the first mentioned method is that the diffusion time and therefore the time between injection and detection is shorter. The advantage of the latter method is that disadvantages associated with the penetration of the inner membrane, like infections, can be reduced.

After the labelled antibody is introduced, it is allowed sufficient time to diffuse through the albumen and migrate to and bind with the selected sex specific antigen on the embryo. The diffusion rate of different labelled antibodies may vary. The skilled man can determine the appropriate diffusion time for a particular selected labelled antibody by measuring the time which is required to get a sufficiently strong detection signal in the measurement of the (relative) concentration of the labelled antibody.

The introduction can be done before or after the start of the incubation. In one embodiment of the method, the labelled antibody is introduced after the egg has been incubated for a time sufficient to allow a sufficient amount of antigens to be generated to be able to bind and detect the labelled antibodies. Typically, detection can take place between 2 and 10 days, preferably between 2 and 7 days more preferably between 2 and 5 days from the start of the incubation. This embodiment has the advantage that introduction and detection can be done in one handling step. In case the diffusion rate of a chosen labelled antibody is relatively slow, the labelled antibody is preferably injected before the start of the incubation to allow sufficient time for the labelled antibody to diffuse through the albumen.

The sex specific antigen on the embryo preferably is W chromosome specific protein antigen, or a ZZ chromosome specific protein antigen, or other indirectly expressed sex specific proteins. Suitable the sex specific antigen on the embryo can be selected from the group of W-specific gene/protein expressions; in particular CHD1W, ATP5A1W, Spin-W, ASW and most preferably FET-1 (female expressed transcript; trans-membrane domain) or of ZZ specific gene/protein expression; in particular DMRT1, SF1 and Dax1, or of the indirectly expressed sex-specific proteins, in particular AMH (Anti-Mullerian hormone).

The antibody can be a polyclonal antibody, a monoclonal antibody or a heavy chain antibody (nanobodies) or a fragment thereof. Heavy chain antibody (nanobodies) and antibody fragments have the advantages that they are smaller and have a higher diffusion rate. The antibodies can be produced on commercial scale in ways known in the art using the selected sex specific antigen.

An antibody can have as a part of its normal composition a functional group capable of detection or can be provided with a detectable functional group in ways known in the art. A functional group preferably is provided to the constant region of the heavy chain, the Fc region (Fragment, crystallizable) of the antibody by binding the luminescent group to the Fc region. It is known in the art of immunofluorescence to label antibodies or antigens with fluorescent dyes. A suitable fluorescent dye is Fluorescein isothiocyanate (FITC), which has excitation and emission wavelengths of approximately 496 nm/521 nm. Like most fluorochromes, it is prone to photobleaching. Because of this newer derivatives of fluorescein such as Alexa 488 and DyLight 488, have been tailored for various chemical and biological applications where greater photostability, higher fluorescence intensity, or different attachment groups are needed. Other suitable and historically common fluorophores are derivatives of rhodamine (TRITC), coumarin, and cyanine. Other suitable detectable functional groups are recombinant proteins containing fluorescent protein domains, e.g. green fluorescent protein (GFP). Use of such "tagged" proteins allows much better localization and less disruption of protein function. Alternatively, the functional group is an enzyme which can be added in combination with a substance that the enzyme can convert to a detectable signal. Thus in the case of fluorescence, when light of the appropriate wavelength is shone upon the sample, any antigen/antibody complexes will fluoresce, with an intensity that is proportional to the amount thereof. The fluorescent signal can be detected by a detector.

The labelled antigen/antibody complexes are detected using detection means positioned outside of the egg. Outside the egg means outside of the inner membrane of the egg or more preferably outside of the entire egg. This is also referred to as non-invasive or in-ovo measurement, as opposed to invasive ex-ovo measurement which requires taking a sample from the egg. Positioning the light source outside the egg and illuminating through the egg shell results is significant light absorption and scattering, but is possible when the eggshell is at least partially transparent for the chosen wavelength. Absorption of light by the eggshell and by membranes is a feature of the egg that can be taken into account in the differential detection technique as described below. Scattering of light can be reduced by using an intense bundled light source and/or by applying a substance on the egg shell having a refractive index close to that of the egg shell.

There are various ways to determine (in-ovo) the presence or absence of binding of the labelled antibodies to the antigen on or near the embryo. The measurement can be based on the fact that the concentration of the labelled antibody will be higher near the embryo because it will (or will not in case of the other sex) bind to the sex specific antigens from the embryo. The position of the blastodisc or the embryo can be determined through the egg shell by optical or acoustic means. When the egg is positioned with the broader end upwards, i.e. with the air cell up (as in Figures 1 - 3), the blastodisc or embryo will move up closer towards the air cell. It is preferred to measure the detection signal from the blastodisc or embryo with the egg in this position through the air cell because there is less light scattering and absorbtion of the light and a better measurement signal can be obtained. In a simple embodiment, illustrated in Fig. 2, the labelled antibody concentration can be detected by measuring a concentration of the labelled antibody or a signal relating to said concentration close to the blastodisc and comparing the measured concentration or signal against a predetermined criterion. The criterion can be predetermined on unfertilised or un-incubated eggs, for any chosen set of measurement conditions including but not limited to a specific choice for the label on the antibody, the chosen antibody/antigen combination, the chosen detection means, for example the wavelength and intensity of detection light, the temperature etc. If the measured signal strength is above a predetermined criterion, the presence of antibody/antigen binding is determined.

In another more reliable method an internal reference measurement is provided in that the presence or absence of binding of the labelled antibodies to the embryo is detected by detecting inhomogeneity in the distribution of the labelled antibodies. The inhomogeneity can be determined by measuring local concentrations of the labelled antibody in a region close to the blastodisc and in a region remote from the blastodisc and determining the presence or absence of binding by comparing the difference or ratio of said measured concentrations against a predetermined criterion, preferably obtained from unfertilised or un-incubated eggs. In a particularly preferred method the region remote from the blastodisc preferably is at the side of the egg yolk opposite to the side where the blastodisc is located. In this way the egg yolk is positioned between the two places of measurement reducing the interference between the two measurements. Another big advantage is that the use of this relative measurement will cancel out the absorption spectrum of the eggshell. So, the result obtained on a white egg will be the same as obtained on a brown version.

In the most preferred embodiment, the antibody is labelled with a marker that is detectable by light, preferably a fluorescent group, and the detection means comprises a light source, a detector and optional filters to improve the signal to noise ratio of absorbed or emitted light, preferably a spectrometer. As illustrated in Figure 1, the detection means (I) preferably comprises a light source (1.3) illuminating the albumen, a first and second detector wherein a first detector (1.2) measures a labelled antibody concentration near the blastodisc, preferably by measuring light intensity of light emitted from the labeled antibody, and wherein a second detector (1.2) measures a labelled antibody concentration in a region remote from the blastodisc, or embryo, preferably at the other side of the egg yolk. Evidently, each detector can be combined with an individual light source as indicated in Figure 3, or more than 2 detectors and/or light sources can be used. Preferably, the wavelength of the detection means is chosen such that the light of the light source and/or of the light emitted by the labelling group on the anti-body is absorbed by the egg yolk to isolate the measurement by the first detector near the blastodisc or embryo from the measurement by the second detector at the other side of the egg yolk and reduce the interference between the two measurements.

Alternatively, the egg is rotated along its length axis during which the concentration of the labelled antibody is measured preferably at a vertical position along its length axis where the blastodisc is positioned. Because the blastodisc and the embryo at early stage of development are not in the centre of the egg and isolated by the light absorbing egg yolk, a higher local concentration of the labelled antibody near the embryo will show up as a peak in the emitted light detection signal. Optionally a reference baseline is subtracted from a measurement of an unfertilised or un-incubated egg.

The light source and/or the detector can also be introduced into the egg through a small opening in the eggshell, preferably in the air cell, but preferably not through the inner shell membrane. This has the advantage of reducing the scattering and absorption of light by the eggshell. This could be done by a very thin fiber optics probe comprising a light source and/or a detector probe and optional filters. The light source can be positioned outside the egg shell and the detector is introduced into the air cell of the egg. Alternatively, only the light source is introduced into the egg and the one or more detectors are positioned outside the egg shell. It is also possible to position the light source and/or the detector outside the egg in an embodiment wherein the wavelength of the excitation light and/or the emission light is not substantially absorbed or scattered by the egg-shell. The wavelength of the excitation light and/or the emission light is preferably selected between 700 and 2000 nm, preferably 700 - 1500, more preferably 700 - 1000 or between 610 and 630 nm. It was found that the egg shell is most transparent at these wavelengths.

Also disclosed is a method and apparatus for in-ovo measurement, i.e. for measuring a labelled molecule in an egg without having to take a sample from the egg. These labelled molecules can be other sex specific molecules than the sex specific labelled antibodies as described herein. For example, it can be another sex specific reagent, for example RNA or DNA sequences to be introduced into the egg or it can be a molecule the egg produces itself, optionally after genetic modification of the egg (or chicken). The labelled molecule can also be another molecule that is introduced in an egg for other purposes, for example a medicament. The molecule is labelled with a functional group capable of in-ovo detection in a way similar as described herein for the labelled antibody. In particular, said method comprises
a. Providing an egg comprising labelled molecules, preferably sex specific molecules, more preferably sex specific antibodies, said molecules having a functional group detectable by in-ovo detection means, preferably a fluorescent or magnetic functional group,
b. measurement of the concentration of the labelled molecules, preferably using a light source and a detector, more preferably a fluorescence spectrometer,
c. evaluating the measured amounts of labelled molecules preferably for determining the sex of an embryo in the egg.

Also disclosed is a an apparatus for pre-hatch determination of the sex of an embryo in an egg according to anyone of the method embodiments described above comprising
a. Means for introducing into the egg a labelled molecule, preferably a labelled antibody, preferably micro-injection means using a needle or micro-fluid injection means using a high-pressure fluid jet,
b. Means for in-ovo measurement of concentration of the labelled molecule, preferably a light source and a detector, more preferably a fluorescence spectrometer.
c. Means for evaluating from the measured amounts of the labelled molecule the presence or absence of binding of the labelled molecule on the embryo.

Preferably, the apparatus comprises a first and second detector wherein the first detector measures a labelled antibody concentration near the blastodisc and wherein the second detector measures a labelled antibody concentration in a region remote from the blastodisc, preferably at the other side of the egg yolk.

Anti-bodies for use in the method according to the invention that are modified with a functional group detectable by ex-ovo measurement, preferably a luminescent group, which anti-bodies preferably are polyclonal antibodies, monoclonal antibodies, heavy chain antibodies or fragments of it wherein the antigen binding side is chosen to match one or more sex specific antigens, preferably chosen from the group W-specific gene/protein expression CHD1W, ATP5A1W, Spin-W, ASW and most preferably FET-1 (female expressed transcript; trans-membrane domain) or of ZZ specific gene/protein expression, in particular DMRT1, SF1 and Dax1, or of the indirectly expressed sex-specific proteins, in particular AMH (Anti-Mullerian hormone).

Anti-Mullerian hormone was chosen as a suitable sex specific hormone. Antibodies for this hormone have already been produced Anti-Mullerian Hormone (antibody 213816, R & D Systems, Minneapolis, Minn.). The antibody is modified with green fluorescent protein (GFP). GFP is not phyto-toxic and is expected to be safe for use in the egg. Suitable techniques modifying the antibody with fluorescent groups are described in Gutowski, M., Carcenac, M., Pourquier, D., Larroque, C., Rouanet, P. and Pèlegrin, A. (2001); "Intraoperative immunophotodetection for radical resection of cancer: evaluation in an experimental model", Clin. Cancer Res. 7, 1142-1148. The labelled antibody is injected into the egg before incubation using micro-injection technique described in WO2006/078499. The egg is positioned with the broad end upwards. After 3 days of incubation the egg is positioned in a measurement cell comprising 2 light sources and 2 detectors (see Figure 3). The light source for exciting the GFP emits light at a wavelength of 395 nm. The GFP emission peak is at 509 nm. The detectors for the emitted light are provided with filters to filter out the excitation light. The primary light source and detector is positioned near the embryo and the secondary (reference) light source and detector are positioned opposite to the embryo such that the egg yolk separates the primary and secondary detector. The ratio of the primary to secondary detection intensities is determined. The ratio of an un-incubated egg is determined and used as a reference measured in exactly the same measurement conditions. The incubated egg shows an increased ratio of the two detection signals compared to the un-incubated egg, indicative of preferential bonding of the labelled anti-body near the blastodisc and of the presence of a male embryo.

## Claims

1. A method for pre-hatch determination of the sex of an embryo in an avian egg, comprising the steps of
a. introducing into the egg an antibody designed to match with a sex specific antigen on the embryo, which antibody is labelled,
b. allowing the labelled antibody to migrate to and bind with the sex specific antigen on the embryo,
c. detecting binding of the labelled antibodies on the embryo using detection means positioned outside of the egg.

2. The method according to claim 1, wherein the labelled antibody is injected into the egg, preferably by micro-injection using a needle or by micro-fluid injection using a high-pressure fluid jet or by osmosis.

3. Method according to claim 1 or 2, wherein the sex specific antigen on the embryo is selected from the group of W-specific gene/protein expression, in particular CHD1W, ATP5A1W, Spin-W, ASW and most preferably FET-1 (female expressed transcript; trans-membrane domain) or of ZZ specific gene/protein expression, in particular DMRT1, SF1 and Dax1, or of the indirectly expressed sex-specific proteins, in particular AMH (Anti-Mullerian hormone).

4. A method according to anyone of claims 1 to 3, wherein detection takes place between 2 and 10 days, preferably between 2 and 7 days more preferably between 2 and 5 days from incubation.

5. The method according to anyone of claims 1 to 4, wherein the antibody is a polyclonal antibody, a monoclonal antibody or a heavy chain antibody or a fragment thereof.

6. The method according to anyone of claims 1 to 5 wherein the presence or absence of binding of the labelled antibodies to the embryo is detected by measuring a concentration of the labelled antibody close to the blastodisc and comparing the measured concentration against a predetermined standard criterion preferably obtained from unfertilised or un-hatched eggs.

7. The method according to anyone of claims 1 to 6, wherein the presence or absence of binding of the labelled antibodies to the embryo is detected by detecting inhomogeneity in the distribution of the labelled antibodies.

8. The method according to claim 7, wherein the inhomogeneity is determined by measuring local concentrations of the labelled antibody in a region close to the blastodisc and in a region remote from the blastodisc and determining the presence or absence of binding by comparing the difference or ratio of said measured concentrations against a predetermined criterion, preferably obtained from unfertilised or un-hatched eggs.

9. The method according to anyone of claims 1 to 8, wherein the antibody is labelled with a marker detectable by light, preferably a fluorescent group.

10. The method according to anyone of claims 1 - 9, wherein the detection means comprises a light source, a detector and optional filters to improve the signal to noise ratio of absorbed or emitted light, preferably a spectrometer.

11. The method according to claim 10, wherein a first detector measures a labelled antibody concentration near the blastodisc and wherein a second detector measures a labelled antibody concentration in a region remote from the blastodisc, preferably at the other side of the egg yolk.

12. The method according to claim 10 or 11, wherein the wavelength of the detection means is chosen such that the light of the light source and/or of the light emitted by the labelling group on the anti-body is absorbed by the egg yolk to isolate measurement by the first detector near the blastodisc from the measurement by the second detector at the other side of the egg yolk.

13. The method according to anyone of claims 10 to 12, wherein the light source and/or the detector are introduced into the egg through a small opening in the eggshell, preferably in the air cell, but preferably not through the inner shell membrane.

14. The method according to anyone of claims 10 - 12, wherein the light source and/or the detector are positioned outside the egg and wherein the wavelength of the excitation light and/or the emission light is not substantially absorbed or scattered by the egg-shell.

15. The method according to claim 14, wherein the wavelength of the excitation light and/or the emission light is selected between 700 and 2000 nm, preferably 700 - 1500, more preferably 700 - 1000 or between 610 and 630 nm.

## Patentansprüche

1. Verfahren zur Bestimmung des Geschlechts eines Embryos in einem Vogelei vor dem Schlüpfen, umfassend die Schritte :
a. Einführen eines Antikörpers, der so gestaltet ist, dass er zu einem geschlechtsspezifischen Antigen auf dem Embryo passt, in das Ei, wobei der Antikörper markiert ist;
b. Wandernlassen des markierten Antikörpers und Bindenlassen desselben an das geschlechtsspezifische Antigen auf dem Embryo;
c. Nachweisen der Bindung der markierten Antikörper auf dem Embryo unter Verwendung von Nachweismitteln, die sich außerhalb des Eies befinden.

2. Verfahren gemäß Anspruch 1, wobei der markierte Antikörper in das Ei injiziert wird, vorzugsweise durch Mikroinjektion mit Hilfe einer Nadel oder durch Mikrofluidinjektion unter Verwendung eines Hochdruckfluidstrahls oder durch Osmose.

3. Verfahren gemäß Anspruch 1 oder 2, wobei das geschlechtsspezifische Antigen auf dem Embryo aus der Gruppe der W-spezifischen Gen/Protein-Expression, insbesondere CHD1W, ATP5A1W, Spin-W, ASW und am meisten bevorzugt FET-1 (weiblich exprimiertes Transcript; Transmembrandomäne), oder ZZ-spezifischen Gen/Protein-Expression, insbesondere DMRT1, SF1 und Dax1, oder der indirekt exprimierten geschlechtsspezifischen Proteine, insbesondere AMH (Anti-Müller-Hormon), ausgewählt ist.

4. Verfahren gemäß einem der Ansprüche 1 bis 3, wobei der Nachweis zwischen 2 und 10 Tagen, vorzugsweise zwischen 2 und 7 Tagen, besonders bevorzugt zwischen 2 und 5 Tagen, nach der Inkubation stattfindet.

5. Verfahren gemäß einem der Ansprüche 1 bis 4, wobei der Antikörper ein polyklonaler Antikörper, ein monoklonaler Antikörper oder eine schwere Kette eines Antikörpers oder ein Fragment davon ist.

6. Verfahren gemäß einem der Ansprüche 1 bis 5, wobei das Vorhandensein oder das Fehlen einer Bindung der markierten Antikörper an den Embryo dadurch nachgewiesen wird, dass man eine Konzentration des markierten Antikörpers in der Nähe der Keimscheibe misst und die gemessene Konzentration mit einem vorbestimmten Standardkriterium vergleicht, das vorzugsweise von unbefruchteten oder nicht geschlüpften Eiern erhalten wurde.

7. Verfahren gemäß einem der Ansprüche 1 bis 6, wobei das Vorhandensein oder das Fehlen einer Bindung der markierten Antikörper an den Embryo dadurch nachgewiesen wird, dass man eine Inhomogenität in der Verteilung der markierten Antikörper nachweist.

8. Verfahren gemäß Anspruch 7, wobei die Inhomogenität dadurch bestimmt wird, dass man lokale Konzentrationen des markierten Antikörpers in einem Bereich in der Nähe der Keimscheibe und in einem von der Keimscheibe entfernten Bereich misst und das Vorhandensein oder das Fehlen von Bindung dadurch bestimmt, dass man die Differenz oder das Verhältnis der gemessenen Konzentrationen mit einem vorbestimmten Kriterium vergleicht, das vorzugsweise von unbefruchteten oder nicht geschlüpften Eiern erhalten wurde.

9. Verfahren gemäß einem der Ansprüche 1 bis 8, wobei der Antikörper mit einem Marker, der durch Licht nachweisbar ist, vorzugsweise einer fluoreszierenden Gruppe, markiert ist.

10. Verfahren gemäß einem der Ansprüche 1 bis 9, wobei das Nachweismittel eine Lichtquelle, einen Detektor und gegebenenfalls Filter zur Verbesserung des Signal-Rausch-Verhältnisses von absorbiertem oder emittiertem Licht umfasst und vorzugsweise ein Spektrometer ist.

11. Verfahren gemäß Anspruch 10, wobei ein erster Detektor eine Konzentration des markierten Antikörpers in der Nähe der Keimscheibe misst und wobei ein zweiter Detektor eine Konzentration des markierten Antikörpers in einem von der Keimscheibe entfernten Bereich, vorzugsweise auf der anderen Seite des Eidotters, misst.

12. Verfahren gemäß Anspruch 10 oder 11, wobei die Wellenlänge des Nachweismittels so gewählt wird, dass das Licht der Lichtquelle und/oder das von der Markierungsgruppe auf dem Antikörper emittierte Licht vom Eidotter absorbiert wird, um die Messung durch den ersten Detektor in der Nähe der Keimscheibe von der Messung durch den zweiten Detektor auf der anderen Seite des Eidotters zu isolieren.

13. Verfahren gemäß einem der Ansprüche 10 bis 12, wobei die Lichtquelle und/oder der Detektor durch eine kleine Öffnung in der Eischale, vorzugsweise in der Luftkammer, aber vorzugsweise nicht durch die innere Schalenhaut hindurch, in das Ei eingeführt werden.

14. Verfahren gemäß einem der Ansprüche 10 bis 12, wobei sich die Lichtquelle und/oder der Detektor außerhalb des Eies befinden und wobei die Wellenlänge des Anregungslichts und/oder des Emissionslichts durch die Eischale im Wesentlichen nicht absorbiert oder gestreut wird.

15. Verfahren gemäß Anspruch 14, wobei die Wellenlänge des Anregungslichts und/oder des Emissionslichts zwischen 700 und 2000 nm, vorzugsweise 700-1500 nm, besonders bevorzugt 700-1000 nm oder zwischen 610 und 630 nm ausgewählt ist.

## Revendications

1. Méthode de détermination avant éclosion du sexe d'un embryon dans un oeuf aviaire, comprenant les étapes suivantes :
a. l'introduction dans l'oeuf d'un anticorps conçu pour correspondre à un antigène spécifique du sexe se trouvant sur l'embryon, l'anticorps étant marqué,
b. le fait de laisser l'anticorps marqué migrer vers l'antigène spécifique du sexe se trouvant sur l'embryon et se lier à l'antigène spécifique du sexe se trouvant sur l'embryon,
c. la détection de la liaison des anticorps marqués sur l'embryon en utilisant un moyen de détection positionné hors de l'oeuf.

2. Méthode selon la revendication 1, dans laquelle l'anticorps marqué est injecté dans l'oeuf, de préférence par micro-injection au moyen d'une aiguille ou par injection microfluidique en utilisant un jet de fluide à haute pression ou par osmose.

3. Méthode selon la revendication 1 ou 2, dans laquelle l'antigène spécifique du sexe se trouvant sur l'embryon est sélectionné dans le groupe de l'expression d'un gène/d'une protéine spécifique de W, en particulier CHD1W, ATP5A1W, Spin-W, ASW et de manière préférée entre toutes FET-1 (female-expressed transcript; domaine trans-membranaire) ou de l'expression d'un gène/d'une protéine spécifique de ZZ, en particulier DMRT1, SF1 et Dax1, ou des protéines spécifiques du sexe exprimées indirectement, en particulier AMH (Anti-Müllerian hormone).

4. Méthode selon l'une quelconque des revendications 1 à 3, dans laquelle la détection a lieu entre les jours 2 et 10, de préférence entre les jours 2 et 7, et de manière davantage préférée entre les jours 2 et 5 à partir de l'incubation.

5. Méthode selon l'une quelconque des revendications 1 à 4, dans laquelle l'anticorps est un anticorps polyclonal, un anticorps monoclonal ou un anticorps à chaîne lourde ou un fragment de ceux-ci.

6. Méthode selon l'une quelconque des revendications 1 à 5, dans laquelle la présence ou l'absence de liaison des anticorps marqués à l'embryon est détectée par la mesure d'une concentration de l'anticorps marqué à proximité du blastodisque et la comparaison de la concentration mesurée à un critère standard prédéterminé obtenu de préférence à partir d'oeufs non fertilisés ou non éclos.

7. Méthode selon l'une quelconque des revendications 1 à 6, dans laquelle la présence ou l'absence de liaison des anticorps marqués à l'embryon est détectée par l'inhomogénéité de la répartition des anticorps marqués.

8. Méthode selon la revendication 7, dans laquelle l'inhomogénéité est déterminée par la mesure des concentrations locales de l'anticorps marqué dans une région proche du blastodisque et dans une région éloignée du blastodisque et la détermination de la présence ou de l'absence de liaison en comparant la différence ou le rapport desdites concentrations mesurées à un critère prédéterminé, obtenu de préférence à partir d'oeufs non fertilisés ou non éclos.

9. Méthode selon l'une quelconque des revendications 1 à 8, dans laquelle l'anticorps est marqué avec un marqueur détectable par la lumière, de préférence un groupe fluorescent.

10. Méthode selon l'une quelconque des revendications 1 à 9, dans laquelle le moyen de détection comprend une source de lumière, un détecteur et des filtres facultatifs pour améliorer le rapport signal sur bruit de la lumière absorbée ou émise, de préférence un spectromètre.

11. Méthode selon la revendication 10, dans laquelle un premier détecteur mesure la concentration en anticorps marqué près du blastodisque et dans laquelle un second détecteur mesure la concentration en anticorps marqué dans une région éloignée du blastodisque, de préférence de l'autre côté du jaune d'oeuf.

12. Méthode selon la revendication 10 ou 11, dans laquelle la longueur d'onde du moyen de détection est choisie de façon que la lumière de la source de lumière et/ou de la lumière émise par le groupe de marquage se trouvant sur l'anticorps soit absorbée par le jaune d'oeuf pour isoler la mesure par le premier détecteur près du blastodisque de la mesure par le second détecteur de l'autre côté du jaune d'oeuf.

13. Méthode selon l'une quelconque des revendications 10 à 12, dans laquelle la source de lumière et/ou le détecteur sont introduits dans l'oeuf par une petite ouverture dans la coquille, de préférence dans la chambre à air, mais de préférence pas à travers la membrane coquillière interne.

14. Méthode selon l'une quelconque des revendications 10 à 12, dans laquelle la source de lumière et/ou le détecteur sont positionnés hors de l'oeuf et dans laquelle la longueur d'onde de la lumière d'excitation et/ou de la lumière d'émission n'est pas absorbée ou diffusée de manière importante par la coquille.

15. Méthode selon la revendication 14, dans laquelle la longueur d'onde de la lumière d'excitation et/ou de la lumière d'émission est sélectionnée entre 700 nm et 2000 nm, de préférence entre 700 nm et 1500 nm, et de manière davantage préférée entre 700 nm et 1000 nm ou entre 610 nm et 630 nm.
